(19) Europäisches Patentamt / European Patent Office / Office européen des brevets

(11) **EP 4 289 459 B1**

(12) **EUROPEAN PATENT SPECIFICATION**

(45) Date of publication and mention
of the grant of the patent:
**02.10.2024 Bulletin 2024/40**

(21) Application number: **22891175.6**

(22) Date of filing: **04.11.2022**

(51) International Patent Classification (IPC):
**A61M 16/00** (2006.01)    **A61M 16/10** (2006.01)

(52) Cooperative Patent Classification (CPC):
**A61M 16/12; A61M 16/026; A61M 16/16;**
A61M 2016/0027; A61M 2016/003;
A61M 2016/1025; A61M 2016/103;
A61M 2202/0208; A61M 2202/0225; A61M 2205/18;
A61M 2230/005; A61M 2230/04; A61M 2230/30;
A61M 2230/42

(86) International application number:
**PCT/CN2022/129965**

(87) International publication number:
**WO 2023/207023 (02.11.2023 Gazette 2023/44)**

(54) **BREATHING APPARATUS WITH CARBON DIOXIDE COMPENSATION FUNCTION**

ATEMGERÄT MIT KOHLENDIOXIDKOMPENSATIONSFUNKTION

APPAREIL RESPIRATOIRE À FONCTION DE COMPENSATION DE DIOXYDE DE CARBONE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **26.04.2022 CN 202210450492**

(43) Date of publication of application:
**13.12.2023 Bulletin 2023/50**

(73) Proprietor: **Guangzhou Landswick Medical
Technologies Ltd.
Guangzhou, Guangdong 510000 (CN)**

(72) Inventors:
• **DONG, Hui**
  **Guangzhou, Guangdong 510000 (CN)**
• **ZHAO, Longchao**
  **Guangzhou, Guangdong 510000 (CN)**
• **SUN, Caixin**
  **Guangzhou, Guangdong 510000 (CN)**

(74) Representative: **Ipside
7-9 Allée Haussmann
33300 Bordeaux Cedex (FR)**

(56) References cited:
CN-A- 106 178 210    CN-A- 111 658 932
CN-A- 112 423 821    CN-A- 113 413 529
CN-A- 113 413 529    CN-A- 114 733 024
CN-U- 204 092 768    JP-A- H0 984 876
US-A1- 2004 144 383  US-A1- 2006 144 396
US-A1- 2020 368 464  US-A1- 2021 052 220
US-A1- 2021 052 220  US-B2- 10 716 912

**Description**

BACKGROUND OF THE PRESENT INVENTION

**Field of Invention**

The present invention relates to the technical field of breathing device, and more particularly to a breathing device with carbon dioxide compensation function.

**Description of Related Arts**

According to the service environment, ventilators are divided into medical ventilators and household ventilators. Medical ventilators are used under the supervision of medical staff for patients with respiratory failure and barotrauma, as well as those requiring respiratory support, respiratory treatment, and first aid resuscitation. The medical ventilators mainly include therapeutic ventilators, first aid ventilators, out-of-hospital transport ventilators, high-frequency jet ventilators, high-frequency oscillation ventilators. Household ventilators are used to relieve snoring, hypopnea and sleep apnea during sleep, moderate and mild respiratory failure, and respiratory insufficiency, thus achieving adjuvant therapeutic purposes. They are commonly used in the family environment, and is also able to be used in medical institutions, and mainly include household respiratory support equipment, sleep ventilators, sleep non-invasive ventilators, continuous positive pressure ventilators, bilevel positive airway pressure ventilators and positive pressure ventilation therapy apparatuses.

Currently, the air source of all ventilators is air and pure oxygen. Existing ventilators are only able to provide patients with mixed air-oxygen, air or pure oxygen, and are unable to provide patients with carbon dioxide gas. Therefore, existing ventilators are unable to compensate for carbon dioxide in the body of the patient by adjusting the proportion of carbon dioxide in the supplied air.

SUMMARY OF THE PRESENT INVENTION

The present invention provides a breathing device with carbon dioxide compensation function, so as to solve the above problems in the prior art.

A breathing device with carbon dioxide compensation function comprises:
an air source pipeline, a low pressure oxygen source pipeline, a high pressure oxygen source pipeline, a carbon dioxide source pipeline, a gas supply pipeline all of which are set in a ventilator; and a breathing pipeline which is set outside the ventilator, wherein:

air from an air source enters the ventilator through the air source pipeline, low pressure oxygen from a low pressure oxygen source is mixed with the air in the air source pipeline through the low pressure oxygen source pipeline; mixed breathing gas enters the gas supply pipeline, is transmitted outside the ventilator through the gas supply pipeline, and is inhaled by a user through the breathing pipeline;

high pressure oxygen from a high pressure oxygen source enters the ventilator through the high pressure oxygen source pipeline, carbon dioxide gas from a carbon dioxide source and the high pressure oxygen in the high pressure oxygen source pipeline are mixed through the carbon dioxide source pipeline and form therapeutic gas; the therapeutic gas enters the gas supply pipeline, is transmitted outside the ventilator through the gas supply pipeline, and is inhaled by the user through the breathing pipeline for treatment, wherein the carbon dioxide gas is mixed with the high pressure oxygen in a preset ratio.

Preferably, a first filter is provided on the air source pipeline, a second filter and a first check valve are provided on the low pressure oxygen source pipeline, the low pressure oxygen flows through the second filer and first check valve in sequence and enters an oxygen flow sensor, the low pressure oxygen and the air from the oxygen flow sensor enter a breathing gas generator through a second check valve, and are mixed by the breathing gas generator for forming breathing gas;
a first pressure sensor is provided on the low pressure oxygen source pipeline and is located between the first check valve and the oxygen flow sensor for measuring a pressure of the low pressure oxygen in the low pressure oxygen source pipeline, the breathing gas enters the gas supply pipeline through a third check valve.

Preferably, a third filter, a first flow control valve, and a directional valve are provided on the high pressure oxygen source pipeline, a second pressure sensor is located between the third filter and the first flow control valve; when the directional valve is opened positively, the high pressure oxygen enters the gas supply pipeline through the third filter, the first flow control valve, and the directional valve on the high pressure oxygen source pipeline;

a fourth filter, a third pressure sensor and a second flow control valve are provided on the carbon dioxide source pipeline in sequence, the carbon dioxide gas and the high pressure oxygen are mixed through the fourth filter, the third pressure sensor and the second flow control valve on the carbon dioxide source pipeline and form therapeutic gas, the therapeutic gas enters the gas supply pipeline;

the third pressure sensor measures a pressure of the carbon dioxide gas in the carbon dioxide source pipeline; a ratio of oxygen to the carbon dioxide gas in the therapeutic gas is adjusted through adjusting a flow of the high pressure oxygen and the carbon dioxide gas by the first and second flow control valves, so as to allow the ratio reaches a preset value.

Preferably, an oxygen sensor, a carbon dioxide sensor, a fourth pressure sensor and a gas flow sensor are provided on the gas supply pipeline;
the oxygen sensor detects an oxygen concentration in the gas supply pipeline, the carbon dioxide sensor detects a carbon dioxide concentration in the gas supply pipeline, the fourth pressure sensor detects a gas concentration in the gas supply pipeline, and the gas flow sensor detects a gas flow in the gas supply pipeline.

Preferably, a humidifier and a nebulizer are provided on a breathing pipeline, a fifth pressure sensor is located between the humidifier and the nebulizer, the breathing gas or the therapeutic gas enters a body of the user through the nebulizer;
atomizing gas of the nebulizer is from the high pressure oxygen in the high pressure oxygen source pipeline, the high pressure oxygen enters an atomizing pipeline through a shut-off valve, and then enters the nebulizer through a first throttle valve for atomizing medication.

Preferably, the breathing gas or the therapeutic gas enters the body of the user through the nebulizer, which specifically comprises:

the breathing gas or the therapeutic gas enters the body of the user through the nebulizer via a mask or a respiratory tube;

when the user exhales gas through the mask or the respiratory tube, the exhaled gas enters an expiratory pipeline, the expiratory pipeline is provided with a diaphragm regulating valve, and the exhaled gas enters an external environment through the diaphragm regulating valve;

the high pressure oxygen source pipeline is connected with a PEEP (positive end-expiratory pressure) regulating pipeline, the PEEP regulating pipeline is provided with a pressure regulating valve, a sixth pressure sensor and a second throttle valve; the sixth pressure sensor is connected with the diaphragm regulating valve for detecting PEEP; a pressure of the diaphragm regulating valve is adjusted by the sixth pressure sensor and the second throttle valve;

the pressure regulating valve is opened to a position according to a preset PEEP value, and diverted high pressure oxygen in the high pressure source pipeline enters the external environment through the second throttle valve.

Preferably, the fifth pressure sensor is connected with an alarm; when the fifth pressure sensor detects that a pressure exceeds a preset range, an alarm instruction is sent to the alarm, and the alarm sends out an abnormal pressure alarm to remind occurrence of gas path blockage or gas leakage.

Preferably, when the directional valve is opened reversely, the high pressure oxygen in the high pressure oxygen source pipeline will not enter the gas supply pipeline.

Preferably, the breathing device further comprises an auxiliary gas source pipeline, the auxiliary gas source pipeline is provided with a fifth filter and the a flow control valve, and a seventh pressure sensor is located between the fifth filter and the third flow control valve;

auxiliary gas from an auxiliary gas source is mixed with the high pressure oxygen and the carbon dioxide gas through the auxiliary gas source pipeline, and mixed gas enters the gas supply pipeline;

the auxiliary gas from the auxiliary gas source comprises carbon dioxide and medical gas used by a medical industry for inhalation by the user.

Preferably, the breathing device further comprises a carbon dioxide flow regulation system which comprises a carbon dioxide monitoring AI (artificial intelligence) module;

the carbon dioxide monitoring AI module detects physiological information of the user in real time, wherein the physiological information comprises blood pressure, ECG (electrocardiogram) parameters, $FiO_2$ (fraction of inspiration oxygen), $FiCO_2$ (fraction of inspiration carbon dioxide), respiratory rate, and respiratory tidal volume; the carbon dioxide monitoring AI module predicts the respiratory state of the user on the physiological information based on comprehensive data analysis AI algorithm, determines a ratio of the carbon dioxide and the oxygen according to the respiratory state, and sets the determined ratio of the carbon dioxide and the oxygen to a preset ratio;

the carbon dioxide flow regulation system further comprises a prediction module, wherein the prediction module, which is connected with the carbon dioxide monitoring AI module for obtaining the physiological information, is configured to determine a first time from starting the ventilator to the user inhaling the therapeutic gas, and a second time from the user inhaling the therapeutic gas into the carbon dioxide monitoring AI module for detection to recovery of the physiological information of the user;

based on the first time, the second time, and the physiological information of the user, a pre-opening time is determined, one pre-opening time is corresponding to one opening condition, wherein the opening condition is when the carbon dioxide gas in the body of the user or exhaled by the user exceeds the preset range, the ventilator is started.

Compared with the prior art, the present invention has some advantages as follows.

The present invention provides the breathing device with carbon dioxide compensation function. The breathing device comprises an air source pipeline, a low pressure oxygen source pipeline, a high pressure oxygen source pipeline, a carbon dioxide source pipeline, a gas supply pipeline all of which are set in a ventilator; and a breathing pipeline which is set outside the ventilator. The air from an air source enters the ventilator through the air source pipeline. The low pressure oxygen from a low pressure oxygen source is mixed with the air in the air source pipeline through the low pressure oxygen source pipeline; mixed breathing gas enters the gas supply pipeline, is transmitted outside the ventilator through the gas supply pipeline, and is inhaled by a user through the breathing pipeline. The high pressure oxygen from a high pressure oxygen source enters the ventilator through the high pressure oxygen source pipeline, carbon dioxide gas from a carbon dioxide source and the high pressure oxygen in the high pressure oxygen source pipeline are mixed through the carbon dioxide source pipeline and form therapeutic gas. the therapeutic gas enters the gas supply pipeline, is transmitted outside the ventilator through the gas supply pipeline, and is inhaled by the user through the breathing pipeline for treatment, wherein the carbon dioxide gas is mixed with the high pressure oxygen in a preset ratio. Moreover, the breathing device further comprises the carbon dioxide flow regulation system for detecting and regulating carbon dioxide in the breathing gas which is transported by the breathing device for patients, so as to compensate for the carbon dioxide in the body of the patient. The carbon dioxide flow control parameters are dynamically adjusted for preventing patients from hyperventilation, and there is no need to collect the carbon dioxide gas exhaled or produced by patients in this process.

Other features and advantages of the present invention will be described in the subsequent specification, and partially become apparent from the specification, or be understood by implement the present invention. Objects and other advantages of the present invention are able to be realized and obtained by the structure specified in the specification, claims, and drawings.

The technical solutions of the present invention are further described in detail by the accompanying drawings and embodiments as follows.

BRIEF DESCRIPTION OF THE DRAWINGS

The present invention is able to be further understood with accompanying drawings. The drawings are a part of the specification and are used to explain the present invention in combination with embodiments as follows, which are not a limitation of the present invention.

Fig. 1 is a structural schematic diagram of a breathing device with carbon dioxide compensation function provided by the present invention.

Fig. 2 is another structural schematic diagram of the breathing device with carbon dioxide compensation function provided by the present invention.

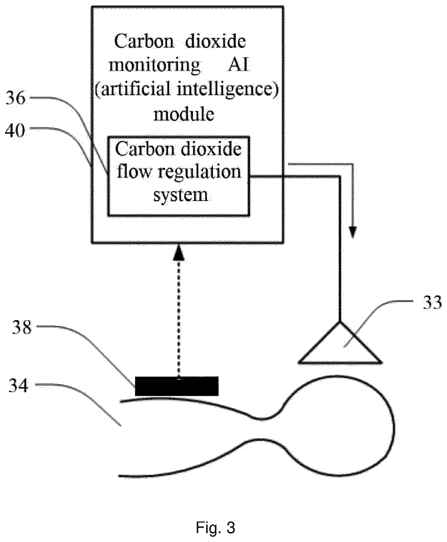

Fig. 3 is a structural schematic diagram of a carbon dioxide flow regulation system.

In the drawings, 1: ventilator; 2: air source; 3: low pressure oxygen source; 4: high pressure oxygen source; 5: carbon dioxide source; 6: auxiliary gas source; 9: first pressure sensor; 10: oxygen flow sensor; 11: breathing gas generator; 12: carbon oxide sensor; 14: first flow control valve; 15: directional valve; 16: second flow control valve; 17: third flow control valve; 18: pressure regulating valve; 19: shut-off valve; 20: sixth pressure sensor; 21: first throttle valve; 22: second throttle valve; 23: external environment; 24: second pressure sensor; 25: third pressure sensor; 26: seventh pressure sensor; 27: oxygen sensor; 28: gas flow sensor; 29: humidifier; 30: fifth pressure sensor; 31: diaphragm regulating valve; 32: nebulizer; 33: mask; 34: user; 35: fourth pressure sensor; 36: carbon dioxide flow regulation system; 38: physiological detection instrument; 40: carbon dioxide monitoring AI (artificial intelligence) module; 108: first check valve; 207: first filter; 208: second check valve; 218: third check valve; 307: second filter; 413: third filter; 513: fourth filter; 613: fifth filter.

DETAILED DESCRIPTION OF THE PREFERRED EMBODIMENT

The present invention will be further described in detail with reference to the accompanying drawings and embodiments. It should be understood that the preferred embodiments described herein are intended only to illustrate and explain the present invention and are not intended to limit the present invention.

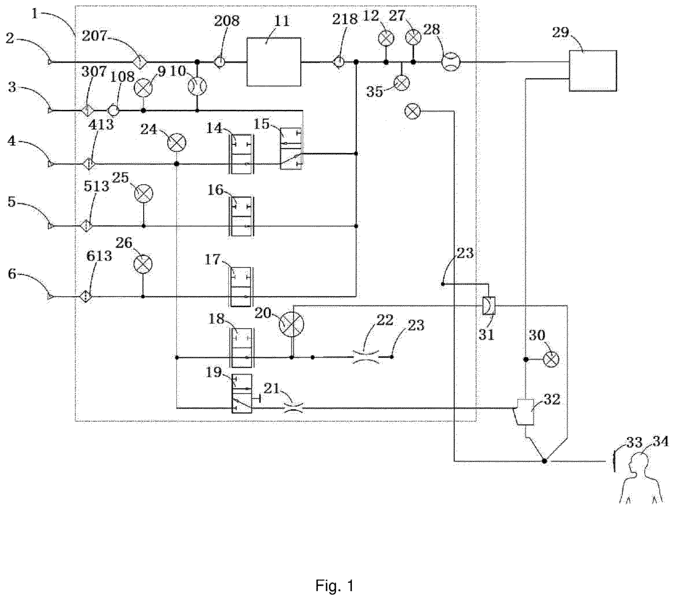

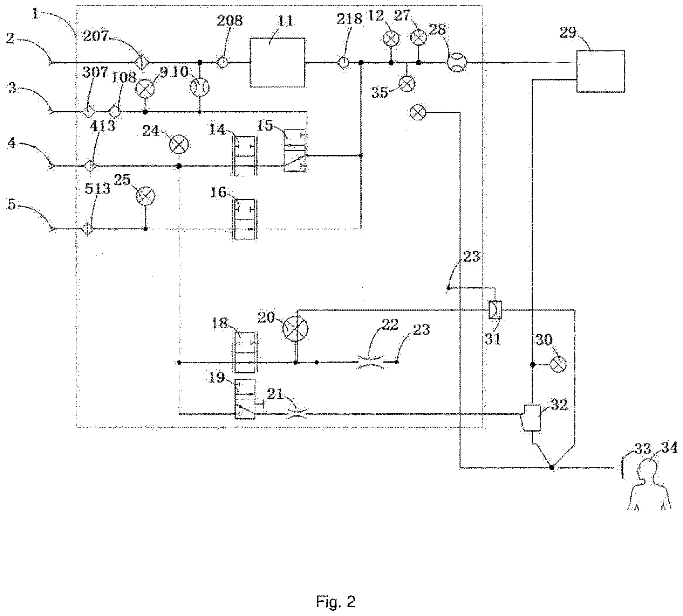

Referring to Figs. 1 and 2, a breathing device with carbon dioxide compensation function is illustrated. The breathing device comprises an air source pipeline, a low pressure oxygen source pipeline, a high pressure oxygen source pipeline, a carbon dioxide source pipeline, a gas supply pipeline all of which are set in a ventilator 1; and a breathing pipeline which is set outside the ventilator 1, wherein:

the air from the air source 2 enters the ventilator 1 through the air source pipeline, the low pressure oxygen from the low pressure oxygen source 3 is mixed with the air in the air source pipeline through the low pressure oxygen source pipeline; the mixed breathing gas enters the gas supply pipeline, is transmitted outside the ventilator 1 through the gas supply pipeline, and is inhaled by a user 34 through the breathing pipeline;

the high pressure oxygen from the high pressure oxygen source 4 enters the ventilator 1 through the high pressure oxygen source pipeline, the carbon dioxide gas from the carbon dioxide source 5 and the high pressure oxygen in the high pressure oxygen source pipeline are mixed through the carbon dioxide source pipeline and form the therapeutic gas; the therapeutic gas enters the gas supply pipeline, is transmitted outside the ventilator 1 through the gas supply pipeline, and is inhaled by the user 34 through the breathing pipeline for treatment, wherein the carbon dioxide gas is mixed with the high pressure oxygen in a preset ratio.

The working principle of the above technical solution is as follows. According to the present invention, there are two breathing control systems in the breathing device, namely, a therapeutic breathing system and an universal breathing system, wherein the two breathing control systems share the same gas supply pipeline; in the therapeutic breathing system, the high pressure oxygen from the high pressure oxygen source 4 and the carbon dioxide gas from the carbon dioxide source 5 are mixed to form the therapeutic gas for the user 34; in the universal breathing system, the low pressure oxygen from the low pressure oxygen source 3 and the air from the air source 2 are mixed to form the breathing gas for the user 34.

Specifically, the breathing device comprises an air source 2, a low pressure oxygen source 3, a high pressure oxygen source 4, a carbon dioxide source 5, a first pressure sensor 9, an oxygen flow sensor 10, a first flow control valve 14, a directional valve 15, a shut-off valve 19, a sixth pressure sensor 20, a first throttle valve 21, a second throttle valve 22, a fifth pressure sensor 30, a diaphragm regulating valve 31, a nebulizer 32, a first check valve 108, a first filter 207, a second filter 307, a third filter 413, a fourth filter 513 and a fifth filter 613.

When the general breathing function is activated, the directional valve 15 is switched and connected with the oxygen flow sensor 10, the high pressure oxygen from the high pressure oxygen source 4 flows through the third filter 413 for entering the ventilator 1, a proportion of the oxygen in the respiratory airflow for the user 34 is set through the first flow control valve 14, the second pressure sensor 24 monitors the pressure of the high pressure oxygen entering the ventilator 1, and the oxygen sensor 27 detects the oxygen concentration. When no high pressure oxygen source 4 is provided, the low pressure oxygen source 3 is provided, the low pressure oxygen from the low pressure oxygen source flows through the first check valve 108 and enters the oxygen flow sensor 10, the first pressure sensor 9 monitors the pressure of the low pressure oxygen, the flow of the low pressure oxygen is determined by the low pressure oxygen source 3 and is generally not higher than 15 L/min. The air from the air source 2 flows through the first filter 207 for entering the ventilator 1, and then is mixed with the oxygen from the oxygen flow sensor 10, and then enters the breathing gas generator 11 through the second check valve 208. The tidal gas generated by the breathing gas generator 11 flows through the third check valve 218 and enters the gas supply pipeline. The oxygen sensor 27 detects the oxygen concentration. The gas flow sensor 28 detects the flow of the tidal gas. The fourth pressure sensor 35 detects the pressure of the gas supply pipeline. The tidal gas flows outside the ventilator 1 and enters the respiratory tube of the user. If the humidity and temperature of the tidal gas need to be adjusted, the humidifier 29 is able to be connected with the ventilator 1 in series. If medication needs to be added to the tidal gas, the breathing pipeline is provided with a nebulizer 32, and at this time, the shut-off valve 19 is switched on, the diverted gas from the high pressure oxygen source 4 flows through the first throttle valve 21 and enters the nebulizer 32 for nebulizing the medication; the tidal gas is inhaled by the user 34 through a mask 33 or the respiratory pipeline. The exhaled gas from the user flows through the diaphragm regulating valve 31 and enters the external environment 23. The pressure of the diaphragm regulating valve 31 is adjusted by the sixth pressure sensor 20 and the second throttle valve 22. While using the PEEP (positive end-expiratory pressure) regulating function, the pressure regulating valve 18 is opened to a certain position according to the preset value of PEEP, the diverted gas from the high pressure oxygen source 4 flows through the second throttle valve 22 and enters the external environment 23; the sixth pressure sensor 20 is connected with the pressure regulating valve 18 and the second throttle valve 22, so the sixth pressure sensor 20 monitors the pressure between the pressure regulating valve 18 and the second throttle valve 22. The sixth pressure sensor 20 is also connected with the diaphragm regulating valve 31, so the pressure of the location where the pressure regulating valve 18, the sixth pressure sensor 20, the second throttle valve 22 and the diaphragm regulating valve 31 are connected is defined as PEEP.

Beneficial effects of the above technical solution are as follows. There is a carbon dioxide flow regulation system 36 in the ventilator 1 for detecting and regulating the carbon dioxide gas in the breathing gas which is delivered to the user by the ventilator 1, so as to compensate for the amount of carbon dioxide or hydrogen ions $H^+$ in the user's body, so that the flow control parameters of the carbon dioxide gas are dynamically adjusted for preventing the user from hyperventilation, and there is no need to collect the carbon dioxide gas exhaled or produced by the user in this process.

Preferably, the first filter 207 is provided on the air source pipeline, that is, the first filter 207 is connected with the air source 2; the second filter 307 and the first check valve 108 are provided on the low pressure oxygen source pipeline, that is, the second filter 307 is located between the low pressure oxygen source 3 and the first check valve 108; the low pressure oxygen flows through the second filer 307 and first check valve 108 in sequence and enters the oxygen flow sensor 10, the low pressure oxygen and the air from the oxygen flow sensor 10 enter the breathing gas generator 11 through the second check valve 208, and are mixed by the breathing gas generator 11 for forming the breathing gas;

the first pressure sensor 9 is provided on the low pressure oxygen source pipeline and is located between the first check valve 108 and the oxygen flow sensor 10 for measuring the pressure of the low pressure oxygen in the low pressure oxygen source pipeline, the breathing gas enters the gas supply pipeline through the third check valve 218.

Preferably, the third filter 413, the first flow control valve 14, and the directional valve 15 are provided on the high pressure oxygen source pipeline, the second pressure sensor 24 is located between the third filter 413 and the first flow control valve 14; when the directional valve 15 is opened positively, the high pressure oxygen enters the gas supply pipeline through the third filter 413, the first flow control valve 14, and the directional valve 15 on the high pressure oxygen source pipeline;

the fourth filter 513, the third pressure sensor 25 and the second flow control valve 16 are provided on the carbon dioxide source pipeline in sequence, the carbon dioxide gas and the high pressure oxygen are mixed through the fourth filter 513, the third pressure sensor 25 and the second flow control valve 16 on the carbon dioxide source pipeline and form the therapeutic gas, the therapeutic gas enters the gas supply pipeline;

the third pressure sensor 25 measures the pressure of the carbon dioxide gas in the carbon dioxide source pipeline; the ratio of the oxygen to the carbon dioxide gas in the therapeutic gas is adjusted through adjusting the flow of the high pressure oxygen and the carbon dioxide gas by the first and second flow control valves 14, 16, so as to allow the ratio reaches reach the preset value.

Preferably, the oxygen sensor 27, the carbon dioxide sensor 12, the fourth pressure sensor 35 and the gas flow sensor 28 are provided on the gas supply pipeline;

the oxygen sensor 27 detects the oxygen concentration in the gas supply pipeline, the carbon dioxide sensor 12 detects the carbon dioxide concentration in the gas supply pipeline, the fourth pressure sensor 35 detects the gas concentration in the gas supply pipeline, and the gas flow sensor 28 detects the gas flow in the gas supply pipeline.

Preferably, the humidifier 29 and the nebulizer 32 are provided on the breathing pipeline, the fifth pressure sensor 30 is located between the humidifier 29 and the nebulizer 32, the breathing gas or the therapeutic gas enters the body of the user 34 through the nebulizer 32;

the atomizing gas of the nebulizer 32 is from the high pressure oxygen in the high pressure oxygen source pipeline, and the high pressure oxygen enters the atomizing pipeline through the shut-off valve 19.

Preferably, the breathing gas or the therapeutic gas enters the body of the user 34 through the nebulizer 32, which specifically comprises:

the breathing gas or the therapeutic gas enters the body of the user 34 through the nebulizer 32 via the mask 33 or the respiratory tube;

when the user 34 exhales gas through the mask 33 or the respiratory tube, the exhaled gas enters the expiratory pipeline, the expiratory pipeline is provided with the diaphragm regulating valve 31, and the exhaled gas enters the external environment 23 through the diaphragm regulating valve 31;

the high pressure oxygen source pipeline is connected with the PEEP regulating pipeline, the PEEP regulating pipeline is provided with the pressure regulating valve 18, the sixth pressure sensor 20 and the second throttle valve 22; the sixth pressure sensor 20 is connected with the diaphragm regulating valve 31 for detecting the PEEP; the pressure of the diaphragm regulating valve 31 is adjusted by the sixth pressure sensor 20 and the second throttle valve 22;

the pressure regulating valve 18 is opened to a position according to the preset PEEP value, and the diverted high pressure oxygen in the high pressure source pipeline enters the external environment through the second throttle valve.

PEEP is the positive end-expiratory pressure for reinflating collapsed alveoli, increasing mean airway pressure, improving oxygenation, and reducing pulmonary edema, but at the same time affecting cardiac blood return and left ventricular afterload, and overcoming increased respiratory work. PEEP is often applied to 1-type respiratory failure represented by ARDS (Acute Respiratory Distress Syndrome).

Preferably, the fifth pressure sensor 30 is connected with an alarm; when the fifth pressure sensor 30 detects that the pressure exceeds the preset range, an alarm instruction is sent to the alarm, and the alarm sends out an abnormal pressure alarm to remind the occurrence of gas path blockage or gas leakage.

Preferably, when the directional valve 15 is opened reversely, the high pressure oxygen in the high pressure oxygen source pipeline will not enter the gas supply pipeline.

Preferably, the breathing device further comprises an auxiliary gas source pipeline, the auxiliary gas source pipeline is provided with the fifth filter 613 and the third flow control valve 17, and the seventh pressure sensor 26 is located between the fifth filter 613 and the third flow control valve 17;

the auxiliary gas from the auxiliary gas source 6 is mixed with the high pressure oxygen and the carbon dioxide gas through the auxiliary gas source pipeline, and the mixed gas enters the gas supply pipeline;

the auxiliary gas from the auxiliary gas source 6 comprises the carbon dioxide gas and the medical gas used by the medical industry for inhalation by the user 34.

Preferably, the breathing device further comprises the carbon dioxide flow regulation system 36 which comprises a carbon dioxide monitoring AI (artificial intelligence) module 40;

the carbon dioxide monitoring AI module 40 detects the physiological information of the user 34 in real time, wherein the physiological information comprises blood pressure, ECG (electrocardiogram) parameters, $FiO_2$ (fraction of inspiration oxygen), $FiCO_2$ (fraction of inspiration carbon dioxide), respiratory rate, and respiratory tidal volume; the carbon dioxide monitoring AI module 40 predicts the respiratory state of the user 34 on the physiological information based on the comprehensive data analysis AI algorithm, determines the ratio of the carbon dioxide and the oxygen according to the respiratory state, and sets the determined ratio of the carbon dioxide and the oxygen to a preset ratio;

the carbon dioxide flow regulation system 36 further comprises a prediction module, wherein the prediction module, which is connected with the carbon dioxide monitoring AI module 40 for obtaining the physiological information, is configured to determine the first time from starting the ventilator 1 to the user 34 inhaling the therapeutic gas, and the second time from the user 34 inhaling the therapeutic gas into the carbon dioxide monitoring AI module 40 for detection to the recovery of the physiological information of the user 34;

based on the first time, the second time, and the physiological information of the user 34, the pre-opening time is determined, one pre-opening time is corresponding to one opening condition, wherein the opening condition is when the carbon dioxide gas in the body of the user 34 or exhaled by the user 34 exceeds the preset range, the ventilator 1 is started.

The working principle of the above technical solution is as follows. The carbon dioxide flow regulation system comprises a high pressure oxygen source 4, a carbon dioxide source 5, a third filter 413, a fourth filter 513, a first pressure sensor 24, a second pressure sensor 25, a first flow control valve 14, a second flow control valve 16, and a fifth pressure sensor 30 which is a pressure sensor close to the user. When the carbon dioxide regulation function is activated, the carbon dioxide gas from the carbon dioxide source 5 flows through the fourth filter 513 and enters the ventilator 1; by adjusting the second flow control valve 16, the flow of the carbon dioxide in the breathing gas for the patient or the user 34 is set. The carbon dioxide sensor 12 detects the concentration of the carbon dioxide gas from the carbon dioxide source 5.

Fig. 2 shows the carbon dioxide monitoring AI module 40 based on the carbon dioxide flow regulation system. The carbon dioxide monitoring AI module 40 drives the carbon dioxide flow regulation system 36 to work, so as to provide required carbon dioxide gas for entering the pipeline located at a rear end of the third check valve 218, and then the carbon dioxide is mixed with other gases and sent to the patient or the user 34 through the mask 33. Herein, the required carbon dioxide gas meets the set parameters which are $PCO_2$ (partial pressure of carbon dioxide), $FiCO_2$ and flow of carbon dioxide. The carbon dioxide monitoring AI module 40 obtains the physiological information (including blood pressure, ECG parameters, $FiO_2$, $FiCO_2$, respiratory rate, and respiratory tidal volume) of the patient through the physiological detection instrument 38 in real time, predicts the respiratory state of the patient on the physiological information based on the comprehensive data analysis AI algorithm, determines the possibility of hyperventilation, respiratory alkalosis, respiratory acidosis, hypoxidosis and other diseases, and according to the determination results, $CO_2$ parameters are dynamically adjusted independently to prevent patients from developing the above symptoms.

When the general respiratory function is activated, the tidal volume VT, the respiratory time ratio I:E, the oxygen ratio $FiO_2$, and the PEEP are set; the appropriate breathing mode is selected according to the state of the patient, and then the ventilator 1 starts to work.

When the therapeutic function is started, the tidal volume VT, the respiratory time ratio I:E, the oxygen ratio $FiO_2$, and the PEEP are set, and then the ventilator 1 starts to work. If the pressure is abnormal at the fifth pressure sensor 30, it indicates the gas path blockage or gas leakage. Medical personnel need to check the state of gas path. The therapeutic gas is a mixture of oxygen and carbon dioxide. The two gases are mixed in a fixed proportion and then injected into the poisoned patient for treatment. Other medical gases are also able to be mixed according to the above method and then injected into patients through continuous ventilation or other customized ventilation modes.

The beneficial effects of the above technical solution are as follows. The $CO_2$ monitoring AI module 40 is able to automatically adjust $FiCO_2$ according to the physiological condition of the patient, which prevents the patient from hyperventilation while regulating $CO_2$. In addition, the pre-opening time is preset, the first time and the second time are calculated, the ratio of carbon dioxide to oxygen is adjusted in advance according to the change rule and trend of carbon dioxide content in the body of the user 34, or the therapeutic function of the ventilator 1 is activated in advance to timely supplement the carbon dioxide gas or reduce the inhalation of carbon dioxide gas for the user 34.

In addition, the oxygen content in the body of the user 34 is able to be measured and calculated. Based on the oxygen content, the preset ratio of carbon dioxide gas to high pressure oxygen is able to be further controlled as follows. The calculation method comprises steps of:

obtaining the first linear relationship between oxygen saturation and the dual wavelength light absorption ratio based on optical absorption measurement method and optical pulse plethysmography, wherein the first linear relationship further comprises a linear relationship between oxygen saturation and body mass indexes of the subject to be detected;

obtaining the second linear relationship between oxygen saturation and the intensity of incident light and detected light passing through the body surface based on the first spectrum signature and the second spectrum signature as well as the intensity of incident light and the detected light passing through the body surface of red light and infrared light;

forming a multiple linear regression model by combining the first linear relationship with the second linear relationship; and

calculating the oxygen saturation based on the multiple linear regression model.

The formula of the first linear relationship is as follows:

$$S_aO_2 = \frac{\varepsilon_{r1}}{\varepsilon_{r1} - \varepsilon_{o1}} - \frac{\varepsilon_{r2}(\varepsilon_{o1}C_{Hb}O_2 + \varepsilon_{r1}C_{Hb})}{(\varepsilon_{r1} - \varepsilon_{o1})(\varepsilon_{o2}C_{Hb}O_2 + \varepsilon_{r2}C_{Hb})} - \alpha\frac{W}{H^2},$$

$$K = \frac{\varepsilon_{o1} C_{Hb} O_2 + \varepsilon_{r1} C_{Hb}}{\varepsilon_{o2} C_{Hb} O_2 + \varepsilon_{r2} C_{Hb},}$$

wherein $S_a O_2$ is oxygen saturation, K is dual wavelength light absorption ratio, $\varepsilon_{o1}$ is oxygenated hemoglobin light absorption coefficient under red light, $\varepsilon_{o2}$ is oxygenated hemoglobin light absorption coefficient under infrared light, $\varepsilon_{r1}$ is reduced hemoglobin light absorption coefficient under red light, $\varepsilon_{r2}$ is reduced hemoglobin light absorption coefficient under infrared light, $C_{Hb} O_2$ is oxygenated hemoglobin light content, $C_{Hb}$ is reduced hemoglobin content, a is empirical coefficient and ranges from 0.1 to 0.3, W is the weight of the object to be detected (unit: Kg); H is the height of the object to be detected (unit: m).

The formula of the second linear relationship is as follows:

$$S_a O_2 = 1 - \frac{A\varepsilon_{o2} - \varepsilon_{o1}}{A\varepsilon_{r2} - \varepsilon_{r1} + \varepsilon_{o1} - A\varepsilon_{o2},}$$

$$A = \left( ln \frac{I_{01}}{I_{test1}} \right) \Big/ \left( ln \frac{I_{02}}{I_{test2}} \right),$$

wherein $S_a O_2$ is oxygen saturation, A is ratio of red light absorption to infrared light absorption, $\varepsilon_{o1}$ is oxygenated hemoglobin light absorption coefficient under red light, $\varepsilon_{o2}$ is oxygenated hemoglobin light absorption coefficient under infrared light, $\varepsilon_{r1}$ is reduced hemoglobin light absorption coefficient under red light, $\varepsilon_{r2}$ is reduced hemoglobin light absorption coefficient under infrared light, $I_{01}$ is the intensity of red light incident light, $I_{02}$ is the intensity of infrared light incident light, $I_{test1}$ is the intensity of light detected after red light passes through the body surface; $I_{test2}$ is the intensity of light detected after infrared light passes through the body surface.

The formula of the formed multiple linear regression model is as follows:

$$Y(S_a O_2) = \beta_0 + \beta_1 \left[ \frac{\varepsilon_{r1}}{\varepsilon_{r1} - \varepsilon_{o1}} - \frac{\varepsilon_{r2}(\varepsilon_{o1} C_{Hb} O_2 + \varepsilon_{r1} C_{Hb})}{(\varepsilon_{r1} - \varepsilon_{o1})(\varepsilon_{o2} C_{Hb} O_2 + \varepsilon_{r2} C_{Hb})} - \alpha \frac{W}{H^2} \right] + \beta_2 \left[ 1 - \frac{A\varepsilon_{o2} - \varepsilon_{o1}}{A\varepsilon_{r2} - \varepsilon_{r1} + \varepsilon_{o1} - A\varepsilon_{o2}} \right]$$

wherin $Y(S_a O_2)$ is oxygen saturation determined based on the multiple linear regression model, and $\beta_0$, $\beta_1$ and $\beta_2$ are the regression coefficients of the multiple linear regression model.

Obviously, those skilled in the art may make various alterations and variants of the present invention without deviating from the scope of the present invention. Thus, if these modifications and variations of the present invention fall within the scope of the claims of the present invention and their equivalents, the present invention is also intended to include these modifications and variations.

## Claims

1.   A breathing device with carbon dioxide compensation function, which comprises:
an air source pipeline, a low pressure oxygen source pipeline, a high pressure oxygen source pipeline, a carbon dioxide source pipeline, a gas supply pipeline all of which are set in a ventilator (1); and a breathing pipeline which is set outside the ventilator (1), wherein:

air from an air source (2) enters the ventilator (1) through the air source pipeline, low pressure oxygen from a low pressure oxygen source (3) is mixed with the air in the air source pipeline through the low pressure oxygen source pipeline; mixed breathing gas enters the gas supply pipeline which is located behind a third check valve (218), is transmitted outside the ventilator (1) through the gas supply pipeline, and is inhaled by a user (34) through the breathing pipeline;

high pressure oxygen from a high pressure oxygen source (4) enters the ventilator (1) through the high pressure oxygen source pipeline, carbon dioxide gas from a carbon dioxide source (5) and the high pressure oxygen in the high pressure oxygen source pipeline are mixed through the carbon dioxide source pipeline and form therapeutic gas; the therapeutic gas enters the gas supply pipeline, is transmitted outside the ventilator (1) through the gas supply pipeline, and is inhaled by the user (4) through the breathing pipeline for treatment, wherein the carbon dioxide gas is mixed with the high pressure oxygen in a preset ratio, so as to adjust a content of carbon dioxide in a body of the user (34);

a third filter (413), a first flow control valve (14), and a directional valve (15) are provided on the high pressure oxygen source pipeline, a second pressure sensor (24) is located between the third filter (413) and the first flow control valve (14); when the directional valve (15) is opened positively, the high pressure oxygen enters the gas supply pipeline through the third filter (413), the first flow control valve (14), and the directional valve (15) on the high pressure oxygen source pipeline;

a fourth filter (513), a third pressure sensor (25) and a second flow control valve (16) are provided on the carbon dioxide source pipeline in sequence, the carbon dioxide gas and the high pressure oxygen are mixed through the fourth filter (513), the third pressure sensor (25) and the second flow control valve (16) on the carbon dioxide source pipeline and form therapeutic gas, the therapeutic gas enters the gas supply pipeline;

the third pressure sensor (25) measures a pressure of the carbon dioxide gas in the carbon dioxide source pipeline; a ratio of oxygen to the carbon dioxide gas in the therapeutic gas is adjusted through adjusting a flow of the high pressure oxygen and the carbon dioxide gas by the first flow control valve (14) and the second flow control valve (16), so as to allow the ratio reaches a preset value.

2. The breathing device with carbon dioxide compensation function according to claim 1, wherein a first filter (207) is provided on the air source pipeline, a second filter (307) and a first check valve (108) are provided on the low pressure oxygen source pipeline, the low pressure oxygen flows through the second filer (307) and first check valve (108) in sequence and enters an oxygen flow sensor (10), the low pressure oxygen and the air from the oxygen flow sensor (10) enter a breathing gas generator (11) through a second check valve (208), and are mixed by the breathing gas generator (11) for forming breathing gas;

a first pressure sensor (9) is provided on the low pressure oxygen source pipeline and is located between the first check valve (108) and the oxygen flow sensor (10) for measuring a pressure of the low pressure oxygen in the low pressure oxygen source pipeline, the breathing gas enters the gas supply pipeline through the third check valve (218).

3. The breathing device with carbon dioxide compensation function according to claim 1, wherein an oxygen sensor (27), a carbon dioxide sensor (12), a fourth pressure sensor (35) and a gas flow sensor (28) are provided on the gas supply pipeline;

the oxygen sensor (27) detects an oxygen concentration in the gas supply pipeline, the carbon dioxide sensor (12) detects a carbon dioxide concentration in the gas supply pipeline, the fourth pressure sensor (35) detects a gas concentration in the gas supply pipeline, and the gas flow sensor (28) detects a gas flow in the gas supply pipeline.

4. The breathing device with carbon dioxide compensation function according to claim 1, wherein a humidifier (29) and a nebulizer (32) are provided on a breathing pipeline, a fifth pressure sensor (30) is located between the humidifier (29) and the nebulizer (32), the breathing gas or the therapeutic gas enters a body of the user (34) through the nebulizer (32);

atomizing gas of the nebulizer (32) is from the high pressure oxygen in the high pressure oxygen source pipeline, the high pressure oxygen enters an atomizing pipeline through a shut-off valve (19), and then enters the nebulizer (32) through a first throttle valve (21) for atomizing medication.

5. The breathing device with carbon dioxide compensation function according to claim 4, wherein the breathing gas or the therapeutic gas enters the body of the user (34) through the nebulizer (32), which specifically comprises:

the breathing gas or the therapeutic gas enters the body of the user (34) through the nebulizer (32) via a mask (33) or a respiratory tube;

when the user (34) exhales gas through the mask (33) or the respiratory tube, the exhaled gas enters an expiratory pipeline, the expiratory pipeline is provided with a diaphragm regulating valve (31), and the exhaled gas enters an external environment (23) through the diaphragm regulating valve (31);

the high pressure oxygen source pipeline is connected with a PEEP (positive end-expiratory pressure) regulating pipeline, the PEEP regulating pipeline is provided with a pressure regulating valve, a sixth pressure sensor (20) and a second throttle valve (22); the sixth pressure sensor (20) is connected with the diaphragm regulating valve (31) for detecting PEEP; a pressure of the diaphragm regulating valve (31) is adjusted by the sixth pressure sensor (20) and the second throttle valve;

the pressure regulating valve is opened to a position according to a preset PEEP value, and diverted high pressure oxygen in the high pressure source pipeline enters the external environment through the second throttle valve.

**6.** The breathing device with carbon dioxide compensation function according to claim 4, wherein the fifth pressure sensor (30) is connected with an alarm; when the fifth pressure sensor (30) detects that a pressure exceeds a preset range, an alarm instruction is sent to the alarm, and the alarm sends out an abnormal pressure alarm to remind occurrence of gas path blockage or gas leakage.

**7.** The breathing device with carbon dioxide compensation function according to claim 1, wherein when the directional valve (15) is opened reversely, the high pressure oxygen in the high pressure oxygen source pipeline will enter the gas supply pipeline from a front end of the second check valve (208) instead of a rear end of the third check valve (218).

**8.** The breathing device with carbon dioxide compensation function according to claim 1, wherein the breathing device further comprises an auxiliary gas source pipeline, the auxiliary gas source pipeline is provided with a fifth filter (613) and the a flow control valve (17), and a seventh pressure sensor (26) is located between the fifth filter (613) and the third flow control valve (17);

auxiliary gas from an auxiliary gas source (6) is mixed with the high pressure oxygen and the carbon dioxide gas through the auxiliary gas source pipeline, and mixed gas enters the gas supply pipeline;
the auxiliary gas from the auxiliary gas source (6) comprises carbon dioxide and medical gas used by a medical industry for inhalation by the user (34).

**9.** The breathing device with carbon dioxide compensation function according to claim 1, wherein the breathing device further comprises a carbon dioxide flow regulation system which comprises a carbon dioxide monitoring AI (artificial intelligence) module;

the carbon dioxide monitoring AI module detects physiological information of the user (34) in real time, wherein the physiological information comprises blood pressure, ECG (electrocardiogram) parameters, $FiO_2$ (fraction of inspiration oxygen), $FiCO_2$ (fraction of inspiration carbon dioxide), respiratory rate, and respiratory tidal volume; the carbon dioxide monitoring AI module predicts the respiratory state of the user (34) on the physiological information based on comprehensive data analysis AI algorithm, determines a ratio of the carbon dioxide and the oxygen according to the respiratory state, and sets the determined ratio of the carbon dioxide and the oxygen to a preset ratio;
the carbon dioxide flow regulation system further comprises a prediction module, wherein the prediction module, which is connected with the carbon dioxide monitoring AI module for obtaining the physiological information, is configured to determine a first time from starting the ventilator (1) to the user (34) inhaling the therapeutic gas, and a second time from the user (34) inhaling the therapeutic gas into the carbon dioxide monitoring AI module for detection to recovery of the physiological information of the user (34);
based on the first time, the second time, and the physiological information of the user (34), a pre-opening time is determined, one pre-opening time is corresponding to one opening condition, wherein the opening condition is when the carbon dioxide gas in the body of the user (34) or exhaled by the user exceeds the preset range, the ventilator (1) is started.

**Patentansprüche**

**1.** Atemvorrichtung mit Kohlendioxid-Kompensationsfunktion, die Folgendes umfasst:
eine Luftquellen-Pipeline, eine Niederdruck-Sauerstoffquellen-Pipeline, eine Hochdruck-Sauerstoffquellen-Pipeline, eine Kohlendioxidquellen-Pipeline, eine Gasversorgungs-Pipeline, die alle in einem Beatmungsgerät (1) eingestellt sind; und eine Atem-Pipeline, die außerhalb des Beatmungsgeräts (1) eingestellt ist, wobei:

Luft aus einer Luftquelle (2) durch die Luftquellen-Pipeline in das Beatmungsgerät (1) eintritt, Niederdruck-Sauerstoff aus einer Niederdruck-Sauerstoffquelle (3) durch die Niederdruck-Sauerstoffquellen-Pipeline mit der Luft in der Luftquellen-Pipeline gemischt wird; gemischtes Atemgas in die Gasversorgungs-Pipeline eintritt, die sich hinter einem dritten Rückschlagventil (218) befindet, durch die Gasversorgungs-Pipeline außerhalb des Beatmungsgeräts (1) übertragen und von einem Benutzer (34) durch die Atem-Pipeline eingeatmet wird;

Hochdruck-Sauerstoff aus einer Hochdruck-Sauerstoffquelle (4) durch die Hochdruck-Sauerstoffquellen-Pipeline in das Beatmungsgerät (1) eintritt, Kohlendioxidgas aus einer Kohlendioxidquelle (5) und der Hochdruck-Sauerstoff in der Hochdruck-Sauerstoffquellen-Pipeline durch die Kohlendioxidquellen-Pipeline gemischt werden und therapeutisches Gas bilden; das therapeutische Gas in die Gasversorgungs-Pipeline eintritt, durch die Gasversorgungs-Pipeline aus dem Beatmungsgerät (1) übertragen wird und vom Benutzer (4) durch die Atem-Pipeline zur Behandlung eingeatmet wird, wobei das Kohlendioxidgas mit dem Hochdruck-Sauerstoff in einem voreingestellten Verhältnis gemischt wird, sodass ein Kohlendioxidgehalt im Körper des Benutzers (34) eingestellt werden kann;

ein drittes Filter (413), ein erstes Ablaufsteuerventil (14) und ein Richtungsventil (15) an der Hochdruck-Sauerstoffquelle-Pipeline vorgesehen sind, ein zweiter Drucksensor (24) sich zwischen dem dritten Filter (413) und dem ersten Ablaufsteuerventil (14) befindet; wenn das Richtungsventil (15) positiv geöffnet wird, der Hochdruck-Sauerstoff durch das dritte Filter (413), das erste Ablaufsteuerventil (14) und das Richtungsventil (15) an der Hochdruck-Sauerstoffquelle-Pipeline in die Gasversorgungs-Pipeline eintritt;

ein vierter Filter (513), ein dritter Drucksensor (25) und ein zweites Ablaufsteuerventil (16) in Sequenz an der Kohlendioxidquellen-Pipeline vorgesehen sind, das Kohlendioxidgas und der Hochdruck-Sauerstoff durch den vierten Filter (513), den dritten Drucksensor (25) und das zweite Ablaufsteuerventil (16) an der Kohlendioxidquellen-Pipeline gemischt werden und therapeutisches Gas bilden, das therapeutische Gas in die Gasversorgungs-Pipeline eintritt;

der dritte Drucksensor (25) einen Druck des Kohlendioxidgases in der Kohlendioxidquellen-Pipeline misst; ein Verhältnis von Sauerstoff zu Kohlendioxidgas im therapeutischen Gas durch Einstellen eines Ablaufs des Hochdruck-Sauerstoffs und des Kohlendioxidgases durch das erste Ablaufsteuerventil (14) und das zweite Ablaufsteuerventil (16) eingestellt wird, sodass das Verhältnis einen voreingestellten Wert erreichen kann.

2. Atemvorrichtung mit Kohlendioxid-Kompensationsfunktion nach Anspruch 1, wobei ein erster Filter (207) an der Luftquellen-Pipeline vorgesehen ist, ein zweiter Filter (307) und ein erstes Rückschlagventil (108) an der Niederdruck-Sauerstoffquellen-Pipeline vorgesehen sind, der Niederdruck-Sauerstoff in Sequenz durch den zweiten Filter (307) und das erste Rückschlagventil (108) strömt und in einen Sauerstoff-Ablaufsensor (10) eintritt, der Niederdruck-Sauerstoff und die Luft aus dem Sauerstoff-Ablaufsensor (10) durch ein zweites Rückschlagventil (208) in einen Atemgasgenerator (11) eintreten und von dem Atemgasgenerator (11) gemischt werden, um Atemgas zu bilden; ein erster Drucksensor (9) an der Niederdruck-Sauerstoffquellen-Pipeline vorgesehen ist und sich zwischen dem ersten Rückschlagventil (108) und dem Sauerstoff-Ablaufsensor (10) befindet, um einen Druck des Niederdruck-Sauerstoffs in der Niederdruck-Sauerstoffquellen-Pipeline zu messen, das Atemgas durch das dritte Rückschlagventil (218) in die Gasversorgungs-Pipeline eintritt.

3. Atemvorrichtung mit Kohlendioxid-Kompensationsfunktion nach Anspruch 1, wobei in der Gasversorgungs-Pipeline ein Sauerstoff-Sensor (27), ein Kohlendioxidsensor (12), ein vierter Drucksensor (35) und ein Gasablaufsensor (28) vorgesehen sind;
der Sauerstoff-Sensor (27) eine Sauerstoff-Konzentration in der Gasversorgungs-Pipeline feststellt, der Kohlendioxidsensor (12) eine Kohlendioxidkonzentration in der Gasversorgungs-Pipeline feststellt, der vierte Drucksensor (35) eine Gaskonzentration in der Gasversorgungs-Pipeline feststellt und der Gasablaufsensor (28) einen Gasablauf in der Gasversorgungs-Pipeline feststellt.

4. Atemvorrichtung mit Kohlendioxid-Kompensationsfunktion nach Anspruch 1, wobei ein Luftbefeuchter (29) und ein Vernebler (32) an einer Atem-Pipeline vorgesehen sind, ein fünfter Drucksensor (30) sich zwischen dem Luftbefeuchter (29) und dem Vernebler (32) befindet, das Atemgas oder das therapeutische Gas durch den Vernebler (32) in einen Körper des Benutzers (34) eintritt;
Zerstäubungsgas des Verneblers (32) aus dem Hochdruck-Sauerstoff in der Hochdruck-Sauerstoffquellen-Pipeline ist, der Hochdruck-Sauerstoff durch ein Absperrventil (19) in eine Zerstäubungs-Pipeline eintritt und dann durch ein erstes Drosselventil (21) in den Vernebler (32) zur Zerstäubung von Medikamenten eintritt.

5. Atemvorrichtung mit Kohlendioxid-Kompensationsfunktion nach Anspruch 4, wobei das Atemgas oder das therapeutische Gas durch den Vernebler (32) in den Körper des Benutzers (34) eintritt, was insbesondere Folgendes umfasst:

das Atemgas oder das therapeutische Gas durch den Vernebler (32) über eine Maske (33) oder ein Beatmungsrohr in den Körper des Benutzers (34) eintritt;
wenn der Benutzer (34) Gas durch die Maske (33) oder das Beatmungsrohr ausatmet, tritt das ausgeatmete Gas in eine exspiratorische Pipeline ein, die exspiratorische Pipeline ist mit einem Membranregulierventil (31) bereitgestellt, und das ausgeatmete Gas tritt durch das Membranregulierventil (31) in eine externe Umgebung (23) ein;

die Hochdruck-Sauerstoffquellen-Pipeline ist mit einer PEEP (positiver endexsiratorischer Druck) regulierenden Pipeline verbunden, die PEEP regulierende Pipeline ist durch ein Druckregulierventil, einen sechsten Drucksensor (20) und ein zweites Drosselventil (22) bereitgestellt; der sechste Drucksensor (20) ist mit dem Membranregulierventil (31) zum Feststellen von PEEP verbunden; ein Druck des Membranregulierventils (31) wird durch den sechsten Drucksensor (20) und das zweite Drosselventil eingestellt;

das Druckregulierventil wird auf eine Position nach einem voreingestellten PEEP-Wert geöffnet, und der abgeleitete Hochdruck-Sauerstoff in der Hochdruck-Quellleitung tritt über das zweite Drosselventil in die äußere Umgebung ein.

6. Atemvorrichtung mit Kohlendioxid-Kompensationsfunktion nach Anspruch 4, wobei der fünfte Drucksensor (30) mit einem Alarm verbunden ist; wenn der fünfte Drucksensor (30) feststellt, dass ein Druck einen voreingestellten Bereich überschreitet, wird eine Alarmanweisung an den Alarm gesendet, und der Alarm sendet einen Alarm bei abnormalem Druck, um an das Vorkommen einer Gaswegblockade oder eines Gasaustritts zu erinnern.

7. Atemvorrichtung mit Kohlendioxid-Kompensationsfunktion nach Anspruch 1, wobei wenn das Richtungsventil (15) in umgekehrter Richtung geöffnet wird, der Hochdruck-Sauerstoff in der Hochdruck-Sauerstoffquellen-Pipeline von einem vorderen Ende des zweiten Rückschlagventils (208) anstatt von einem hinteren Ende des dritten Rückschlagventils (218) in die Gasversorgungs-Pipeline eintritt.

8. Atemvorrichtung mit Kohlendioxid-Kompensationsfunktion nach Anspruch 1, wobei die Atemvorrichtung ferner eine Hilfsgasquellen-Pipeline umfasst, die Hilfsgasquellen-Pipeline mit einem fünften Filter (613) und einem Ablaufsteuerventil (17) versehen ist und ein siebter Drucksensor (26) sich zwischen dem fünften Filter (613) und dem dritten Ablaufsteuerventil (17) befindet;

Hilfsgas aus einer Hilfsgasquelle (6) mit dem Hochdruck-Sauerstoff und dem Kohlendioxidgas durch die Hilfsgasquellen-Pipeline gemischt wird und das gemischte Gas in die Gasversorgungs-Pipeline eintritt;

das Hilfsgas aus der Hilfsgasquelle (6) Kohlendioxidgas und medizinisches Gas umfasst, das von einer medizinischen Industrie zum Einatmen durch den Benutzer (34) verwendet wird.

9. Atemvorrichtung mit Kohlendioxid-Kompensationsfunktion nach Anspruch 1, wobei die Atemvorrichtung ferner ein System zum Regulieren des Kohlendioxidablaufs umfasst, das ein Modul zum Überwachen des Kohlendioxidablaufs mit KI (künstlicher Intelligenz) umfasst;

das KI-Modul zum Überwachen des Kohlendioxids physiologische Informationen des Benutzers (34) in Echtzeit feststellt, wobei die physiologischen Informationen Blutdruck, EKG-Parameter (Elektrokardiogramm), $FiO_2$ (Anteil des eingeatmeten Sauerstoffs), $FiCO_2$ (Anteil des eingeatmeten Kohlendioxids), Atemfrequenz und Atemzugvolumen umfassen; das Modul zum Überwachen des Kohlendioxids den Atemzustand des Benutzers (34) auf den physiologischen Informationen basierend auf einem umfassenden KI-Algorithmus zur Datenanalyse vorhersagt, ein Verhältnis des Kohlendioxids und des Sauerstoffs nach dem Atemzustand bestimmt und das bestimmte Verhältnis des Kohlendioxids und des Sauerstoffs auf ein vorgegebenes Verhältnis einstellt;

das System zum Regulieren des Kohlendioxidablaufs ferner ein Vorhersagemodul umfasst, wobei das Vorhersagemodul, das mit dem KI-Modul zum Überwachen des Kohlendioxidgases zum Ermitteln der physiologischen Informationen verbunden ist, konfiguriert ist, einen ersten Zeitpunkt vom Starten des Beatmungsgeräts (1) bis zum Einatmen des therapeutischen Gases durch den Benutzer (34) und einen zweiten Zeitpunkt vom Einatmen des therapeutischen Gases durch den Benutzer (34) in das KI-Modul zum Überwachen des Kohlendioxids zur Erkennung bis zur Wiederherstellung der physiologischen Informationen des Benutzers (34) zu bestimmen;

basierend auf dem ersten Zeitpunkt, dem zweiten Zeitpunkt und den physiologischen Informationen des Benutzers (34) ein Voröffnungszeitpunkt bestimmt wird, wobei ein Voröffnungszeitpunkt einem Öffnungszustand entspricht, wenn das Kohlendioxidgas im Körper des Benutzers (34) oder das vom Benutzer ausgeatmete Gas den voreingestellten Bereich überschreitet, wird das Beatmungsgerät (1) gestartet.

**Revendications**

1. Dispositif respiratoire avec fonction de compensation de dioxyde de carbone, qui comprend :
une canalisation de source d'air, une canalisation de source d'oxygène à basse pression, une canalisation de source d'oxygène à haute pression, une canalisation de source de dioxyde de carbone, une canalisation d'alimentation en gaz qui sont toutes établies dans un ventilateur (1) ; et une canalisation respiratoire qui est établie à l'extérieur du ventilateur (1), dans lequel :

de l'air provenant d'une source d'air (2) pénètre dans le ventilateur (1) par l'intermédiaire de la canalisation de source d'air, de l'oxygène à basse pression provenant d'une source d'oxygène à basse pression (3) est mélangé à l'air dans la canalisation de source d'air à travers la canalisation de source d'oxygène à basse pression ; un gaz respiratoire mélangé pénètre dans la canalisation d'alimentation en gaz qui est située derrière une troisième vanne de retenue (218), est transmis à l'extérieur du ventilateur (1) par l'intermédiaire de la canalisation d'alimentation en gaz, et est inhalé par un utilisateur (34) à travers la canalisation respiratoire ;

de l'oxygène à haute pression provenant d'une source d'oxygène à haute pression (4) pénètre dans le ventilateur (1) par l'intermédiaire de la canalisation de source d'oxygène à haute pression, du dioxyde de carbone gazeux provenant d'une source de dioxyde de carbone (5) et l'oxygène à haute pression dans la canalisation de source d'oxygène à haute pression sont mélangés à travers la canalisation de source de dioxyde de carbone et forment un gaz thérapeutique ; le gaz thérapeutique pénètre dans la canalisation d'alimentation en gaz, est transmis à l'extérieur du ventilateur (1) par l'intermédiaire de la canalisation d'alimentation en gaz et est inhalé par l'utilisateur (4) à travers la canalisation respiratoire pour le traitement, dans lequel le dioxyde de carbone gazeux est mélangé à l'oxygène à haute pression dans un rapport préfixé, de façon à ajuster la teneur en dioxyde de carbone dans le corps de l'utilisateur (34) ;

un troisième filtre (413), une première vanne de contrôle de débit (14) et une vanne directionnelle (15) sont prévus sur la canalisation de source d'oxygène à haute pression, un deuxième capteur de pression (24) est situé entre le troisième filtre (413) et la première vanne de contrôle de débit (14) ; lorsque la vanne directionnelle (15) est ouverte positivement, l'oxygène à haute pression pénètre dans la canalisation d'alimentation en gaz par l'intermédiaire du troisième filtre (413), de la première vanne de contrôle de débit (14) et de la vanne directionnelle (15) sur la canalisation de source d'oxygène à haute pression ;

un quatrième filtre (513), un troisième capteur de pression (25) et une deuxième vanne de contrôle de débit (16) sont prévus sur la canalisation de source de dioxyde de carbone dans l'ordre, le dioxyde de carbone gazeux et l'oxygène à haute pression sont mélangés à travers le quatrième filtre (513), le troisième capteur de pression (25) et la deuxième vanne de contrôle de débit (16) sur la canalisation de source de dioxyde de carbone et forment un gaz thérapeutique, le gaz thérapeutique pénètre dans la canalisation d'alimentation en gaz ;

le troisième capteur de pression (25) mesure une pression du dioxyde de carbone gazeux dans la canalisation de source de dioxyde de carbone ; un rapport entre l'oxygène et le dioxyde de carbone gazeux dans le gaz thérapeutique est ajusté en ajustant un débit de l'oxygène à haute pression et du dioxyde de carbone gazeux par la première vanne de contrôle de débit (14) et la deuxième vanne de contrôle de débit (16), de façon à permettre au rapport d'atteindre une valeur préfixée.

2. Dispositif respiratoire avec fonction de compensation de dioxyde de carbone selon la revendication 1, dans lequel un premier filtre (207) est prévu sur la canalisation de source d'air, un deuxième filtre (307) et une première vanne de retenue (108) sont prévus sur la canalisation de source d'oxygène à basse pression, l'oxygène à basse pression s'écoule à travers le deuxième filtre (307) et la première vanne de retenue (108) dans l'ordre et pénètre dans un capteur de débit d'oxygène (10), l'oxygène à basse pression et l'air provenant du capteur de débit d'oxygène (10) pénètrent dans un générateur de gaz respiratoire (11) par l'intermédiaire d'une deuxième vanne de retenue (208), et sont mélangés par le générateur de gaz respiratoire (11) pour former un gaz respiratoire ;

un premier capteur de pression (9) est prévu sur la canalisation de source d'oxygène à basse pression, et est situé entre la première vanne de retenue (108) et le capteur de débit d'oxygène (10), pour mesurer une pression de l'oxygène à basse pression dans la canalisation de source d'oxygène à basse pression, le gaz respiratoire pénètre dans la canalisation d'alimentation en gaz par l'intermédiaire de la troisième vanne de retenue (218).

3. Dispositif respiratoire avec fonction de compensation de dioxyde de carbone selon la revendication 1, dans lequel un capteur d'oxygène (27), un capteur de dioxyde de carbone (12), un quatrième capteur de pression (35) et un capteur de débit de gaz (28) sont prévus sur la canalisation d'alimentation en gaz ;

le capteur d'oxygène (27) détecte une concentration d'oxygène dans la canalisation d'alimentation en gaz, le capteur de dioxyde de carbone (12) détecte une concentration de dioxyde de carbone dans la canalisation d'alimentation en gaz, le quatrième capteur de pression (35) détecte une concentration de gaz dans la canalisation d'alimentation en gaz, et le capteur de débit de gaz (28) détecte un débit de gaz dans la canalisation d'alimentation en gaz.

4. Dispositif respiratoire avec fonction de compensation de dioxyde de carbone selon la revendication 1, dans lequel un humidificateur (29) et un nébuliseur (32) sont prévus sur une canalisation respiratoire, un cinquième capteur de pression (30) est situé entre l'humidificateur (29) et le nébuliseur (32), le gaz respiratoire ou le gaz thérapeutique pénètre dans le corps de l'utilisateur (34) par l'intermédiaire du nébuliseur (32) ;

un gaz d'atomisation du nébuliseur (32) provient de l'oxygène à haute pression dans la canalisation de source d'oxygène à haute pression, l'oxygène à haute pression pénètre dans une canalisation d'atomisation par l'intermédiaire d'une vanne d'arrêt (19), puis pénètre dans le nébuliseur (32) par l'intermédiaire d'une première vanne d'étranglement (21) pour atomiser une médication.

**5.** Dispositif respiratoire avec fonction de compensation de dioxyde de carbone selon la revendication 4, dans lequel le gaz respiratoire ou le gaz thérapeutique pénètre dans le corps de l'utilisateur (34) par l'intermédiaire du nébuliseur (32), ce qui comprend spécifiquement :

le gaz respiratoire ou le gaz thérapeutique pénètre dans le corps de l'utilisateur (34) par l'intermédiaire du nébuliseur (32) via un masque (33) ou un tube respiratoire ;
lorsque l'utilisateur (34) expire un gaz à travers le masque (33) ou le tube respiratoire, le gaz expiré pénètre dans une canalisation d'expiration, la canalisation d'expiration est dotée d'une vanne de régulation de membrane (31), et le gaz expiré pénètre dans un environnement externe (23) par l'intermédiaire de la vanne de régulation de membrane (31) ;
la canalisation de source d'oxygène à haute pression est raccordée à une canalisation de régulation PEEP (pression positive de fin d'expiration), la canalisation de régulation PEEP est dotée d'une vanne de régulation de pression, d'un sixième capteur de pression (20) et d'une deuxième vanne d'étranglement (22) ; le sixième capteur de pression (20) est raccordé à la vanne de régulation de membrane (31) pour détecter la PEEP ; une pression de la vanne de régulation de membrane (31) est ajustée par le sixième capteur de pression (20) et par la deuxième vanne d'étranglement ;
la vanne de régulation de pression débouche sur une position selon une valeur PEEP préfixée, et de l'oxygène à haute pression détourné dans la canalisation de source à haute pression pénètre dans l'environnement externe par l'intermédiaire de la deuxième vanne d'étranglement.

**6.** Dispositif respiratoire avec fonction de compensation de dioxyde de carbone selon la revendication 4, dans lequel le cinquième capteur de pression (30) est raccordé à une alarme ; lorsque le cinquième capteur de pression (30) détecte qu'une pression dépasse une plage préfixée, une instruction d'alarme est envoyée à l'alarme, et l'alarme envoie une alarme de pression anormale pour rappeler l'occurrence d'un blocage de voie de gaz ou d'une fuite de gaz.

**7.** Dispositif respiratoire avec fonction de compensation de dioxyde de carbone selon la revendication 1, dans lequel, lorsque la vanne directionnelle (15) est ouverte en sens inverse, l'oxygène à haute pression dans la canalisation de source d'oxygène à haute pression pénétrera dans la canalisation d'alimentation en gaz à partir d'une extrémité avant de la deuxième vanne de retenue (208) au lieu d'une extrémité arrière de la troisième vanne de retenue (218).

**8.** Dispositif respiratoire avec fonction de compensation de dioxyde de carbone selon la revendication 1, dans lequel le dispositif respiratoire comprend en outre une canalisation de source de gaz auxiliaire, la canalisation de source de gaz auxiliaire est dotée d'un cinquième filtre (613) et de la/d'une vanne de contrôle de débit (17), et un septième capteur de pression (26) est situé entre le cinquième filtre (613) et la troisième vanne de contrôle de débit (17) ;

un gaz auxiliaire provenant d'une source de gaz auxiliaire (6) est mélangé à l'oxygène à haute pression et au dioxyde de carbone gazeux à travers la canalisation de source de gaz auxiliaire, et le gaz mélangé pénètre dans la canalisation d'alimentation en gaz ;
le gaz auxiliaire provenant de la source de gaz auxiliaire (6) comprend du dioxyde de carbone et du gaz médical utilisé par une industrie médicale pour inhalation par l'utilisateur (34).

**9.** Dispositif respiratoire avec fonction de compensation de dioxyde de carbone selon la revendication 1, dans lequel le dispositif respiratoire comprend en outre un système de régulation de débit de dioxyde de carbone qui comprend un module IA (intelligence artificielle) de surveillance de dioxyde de carbone ;

le module IA de surveillance de dioxyde de carbone détecte des informations physiologiques de l'utilisateur (34) en temps réel, dans lequel les informations physiologiques comprennent la pression artérielle, les paramètres ECG (électrocardiogramme), la $FiO_2$ (fraction d'oxygène à l'inspiration), la $FiCO_2$ (fraction de dioxyde de carbone à l'inspiration), la fréquence respiratoire et le volume courant respiratoire ; le module IA de surveillance de dioxyde de carbone prédit l'état respiratoire de l'utilisateur (34) d'après les informations physiologiques sur la base d'un algorithme IA d'analyse de données complète, détermine un rapport entre le dioxyde de carbone et l'oxygène selon l'état respiratoire, et fixe le rapport déterminé entre le dioxyde de carbone et l'oxygène à un rapport préfixé ;

le système de régulation de débit de dioxyde de carbone comprend en outre un module de prédiction, dans lequel le module de prédiction, qui est raccordé au module IA de surveillance de dioxyde de carbone pour obtenir les informations physiologiques, est configuré pour déterminer un premier temps depuis le démarrage du ventilateur (1) jusqu'à l'inhalation du gaz thérapeutique par l'utilisateur (34), et un deuxième temps depuis l'inhalation du gaz thérapeutique par l'utilisateur (34) dans le module IA de surveillance de dioxyde de carbone pour détection jusqu'à la récupération des informations physiologiques de l'utilisateur (34) ;

sur la base du premier temps, du deuxième temps et des informations physiologiques de l'utilisateur (34), un temps de préouverture est déterminé, un temps de préouverture correspond à une condition d'ouverture, dans lequel la condition d'ouverture est lorsque le dioxyde de carbone gazeux dans le corps de l'utilisateur (34) ou expiré par l'utilisateur dépasse la plage préfixée, le ventilateur (1) est démarré.

Fig. 1

Fig. 2

Fig. 3